# FASCICULE DE BREVET EUROPEEN

(11) **EP 1 191 962 B1**
(45) Date de publication et mention de la délivrance du brevet: **10.11.2004**
(21) Numéro de dépôt: 00920830.7
(22) Date de dépôt: 18.04.2000
(51) Int. Cl.: A61M 5/14

(54) **DISPOSITIF PERMETTANT L'INJECTION DE LA TOTALITE DE PRODUITS ANTIMITOTIQUES, ANTIBIOTIQUES OU PLAQUETTES SANGUINES LORS DE LEUR ADMINISTRATION EN INTRAVEINEUSE LENTE**
VORRICHTUNG ZUM INJIZIEREN VON ALLEN ANTIMITOTISCHEN, ANTIBIOTISCHEN PRODUKTEN ODER BLUTPLÄTTCHEN WÄHREND IHRER LANGSAMEN INTRAVENÖSEN VERABREICHUNG
DEVICE FOR COMPLETE INJECTION OF ANTIMITOTIC, ANTIBIOTIC PRODUCTS OR BLOOD PLATELETS WHEN ADMINISTERED BY SLOW INTRAVENOUS PERFUSION

(30) Priorité: 10.05.1999 FR 9906135
(43) Date de publication de la demande: 03.04.2002
(73) Titulaire: Reifi Ltd, Dublin 2 (IE)
(72) Inventeur: Gautier, Jean, F-35000 Rennes (FR)
(74) Mandataire: Geismar, Thierry
(86) Numéro de dépôt international: PCT/FR2000/001006
(87) Numéro de publication internationale: WO 2000/067823

(56) Documents cités:
- WO-A-92/08503
- US-A- 2 784 733
- US-A- 4 055 176
- US-A- 4 865 583
- US-A- 5 439 452
- US-A- 5 527 295

## Description

La présente invention concerne un dispositif destiné à permettre l'injection de la totalité de produits antimitotiques, antibiotiques ou de plaquettes de sang lors de leur administration en intraveineuse lente. Ceci, sans résidu dans la tubulure et en parfaite sécurité pour le patient et le personnel médical.

Il est en effet établi par les praticiens, d'une part, qu'avec le système traditionnel de perfusion par gravité, simple et peu onéreux, composé d'une poche plastique, d'un perforateur, d'une chambre goutte à goutte avec filtre et d'un régulateur manuel, que la totalité du produit n'est pas administrée. Ce système par gravité est généralement utilisé, en dehors d'autres systèmes plus rares et beaucoup plus onéreux dits pousse seringue, perfuseur spécifique aux transfusions sanguines ou pompe à perfusion. Lorsque la poche contenant le produit actif est vide, il reste, en moyenne, 15 millilitres de produit actif dans la tubulure qui sont souvent jetés avec cette tubulure. Ce qui, sur une perfusion d'un millilitre de produit anticancéreux actif, dilué dans 50 millilitres de produit neutre, de type glucose ou NACL, le patient n'est traité qu'à 70%. Et que pour des antibiotiques, un tel sous traitement peut provoquer des résistances.

Il faut arriver à injecter l'intégralité du produit actif contenu dans la tubulure, par l'adjonction d'un rinçage, qui, en premier, purge la tubulure de son produit actif et, en second, nettoie la veine de ses résidus actifs, tout en permettant l'éventualité d'une autre perfusion d'antimitotique sans difficulté d'incompatibilité avec le premier antimitotique. Et ce sans désolidariser le perforateur de la poche initiale afin d'assurer une sécurité absolue des personnes en raison d'émission d'aérosols ou de projection de liquide actif.

Un certain nombre de règles de sécurité s'imposent. D'une part, il est interdit d'administrer un liquide de rinçage par simple ajout d'injection par aiguille dans le site d'injection complémentaire équipant toutes les poches existantes. D'autre part, le retrait de l'aiguille et sa désolidarisation avec la seringue comportent un danger pour le manipulateur et le patient.

On connait du document US-5439452 un dispositif selon le préambule de la revendication 1.

Le dispositif comporte
- une chambre d'air, dite de goutte à goutte, de préférence de type traditionnel en plastique translucide et souple, sur laquelle est soudée, directement, en son sommet, entre le perforateur destiné à pénétrer dans la poche de produit et la chambre goutte à goutte, une robinetterie à trois voies, dont l'une est obligatoirement à pas de vis clampé pour y adjoindre de manière définitive la seringue de rinçage, elle aussi à pas de vis. Ceci, afin d'éviter, comme expliqué plus haut, l'utilisation et la manipulation avec aiguille. L'absence d'aiguille permet d'ailleurs à l'infirmière, une fois l'injection du produit actif et le rinçage effectués, de jeter l'ensemble sans le désolidariser. Et donc, d'éviter toute émission d'aérosols d'antimitotique, dangereux pour la santé et l'environnement. Sans parler des risques de piqûres accidentelles de préférence;
- au tiers de la hauteur de la chambre d'air est encastré ou soudé un filtre à résidu.

Le dispositif comporte également, dans la partie inférieure de la chambre d'air:
- d'une part, l'obturation de l'évacuation basse du liquide au moyen d'une bille creuse, de préférence en plastique, flottante. Cette bille est canalisée dans son déplacement vertical par une colonne creuse. Cette colonne creuse, de préférence en plastique dur et translucide est perforée en plusieurs endroits pour permettre la circulation du liquide.
- d'autre part, la base de la chambre d'air, traditionnellement plate, est ici conique pour éviter un trop important reste de produit actif en attendant le rinçage.

Les dessins annexés illustrent un mode de réalisation de l'invention:
La figure 1 représente en coupe, le dispositif général.
La figure 2 représente en coupe, un agrandissement de la seconde partie du dispositif de l'invention au niveau de l'évacuation du produit

En référence à ces dessins,

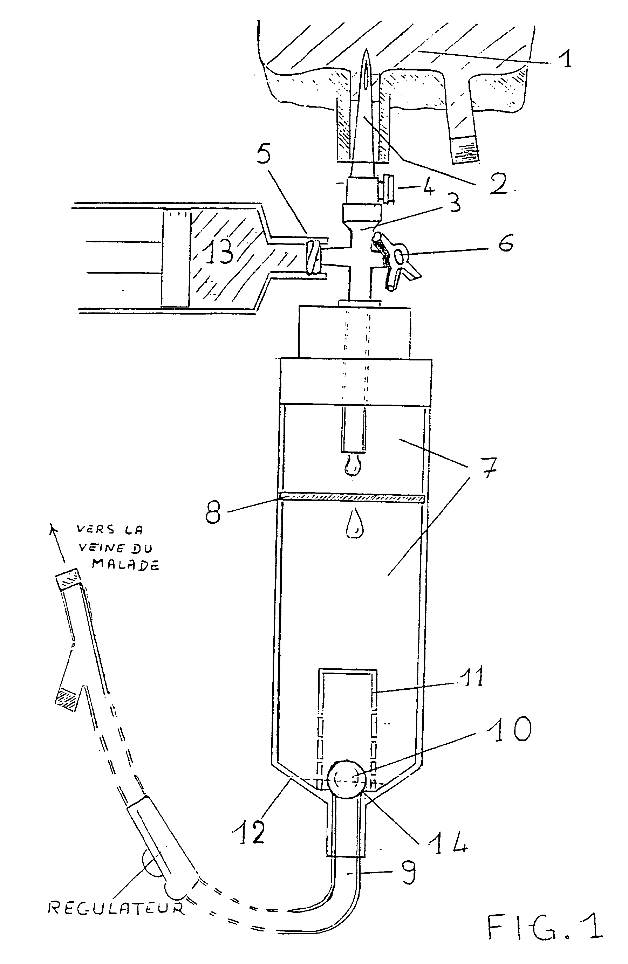

**Figure 1** le dispositif comporte en son sommet, le perforateur de la poche contenant le liquide actif à injecter (1). La partie basse de ce perforateur est soudée à la voie supérieure (2) d'un robinet à trois voies (3). Ce robinet à trois voies est lui même soudé au sommet de la chambre d'air de goutte à goutte (7). La partie latérale, à pas de vis clampé (5), est appelée à recevoir, sans permettre de l'en désolidariser ensuite, la seringue à pas de vis destinée à l'injection du liquide de rinçage (13).Une manette de robinet (6) assure, lors de l'opération «perfusion du produit actif», le transfert du liquide, vers la chambre d'air, puis lors de l'opération « rinçage », dans , un premier temps, de la seringue vers la poche plastique (1), et dans un second temps, de la poche plastique (1) vers la chambre d'air (7). Au tiers haut de la chambre d'air (7) est serti un filtre à résidus(8). Au fond de la chambre d'air (7), une bille (10) canalisée dans un tube (11) perforé, vient obturer l'orifice (14) de départ vers la tubulure d'injection (9).

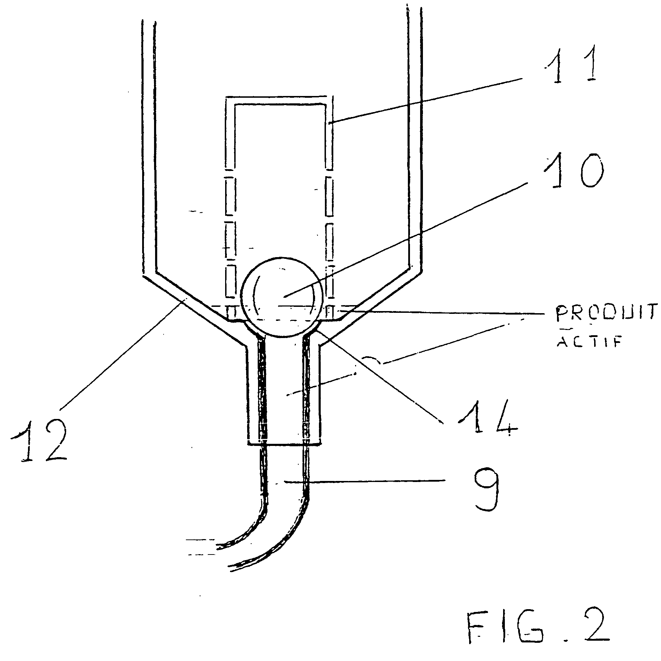

**Figure 2** la vue agrandie du dispositif montre que le fond de la chambre d'air (7) est conique selon une forme d'entonnoir (12) et relié à la tubulure d'injection (9). Cette tubulure d'injection (9) dépasse d'un ou deux millimètres la base interne de l'entonnoir pour permettre à la bille flottante(10), mais d'un poids suffisant, de venir se poser sur le sommet de la tubulure d'injection (9), sans toucher aux parois, et de l'obturer parfaitement Une colonne perforée (11) guide la bille (10) vers le sommet de la tubulure d'injection (9). En effet, cette colonne (11), soudée sur la base du cône, et dont le diamètre est supérieur de deux millimètres à celui de la bille, vient couvrir l'orifice d'évacuation 14. Grace à un niveau résiduel d'un minimum de liquide, dû à la forme conique (12), au poids de la bille et à l'aspiration par gravité, la bille (10) est bloquée sur le sommet de l'orifice de sortie, et le passage d'air dans la tubulure est stoppé. L'assise prévue pour la bille (10) doit être d'un diamètre suffisant pour permettre l'enfoncement de celle-ci sur environ le tiers de son diamètre. Cette assise doit épouser la forme arrondie de la bille pour une parfaite étanchéité. Lorsque l'adjonction du produit de rinçage (13) est ensuite effectuée au moyen de la seringue évoquée ci-dessus, la bille (10), se remet à flotter dans sa colonne de guidage (11) au niveau voulu. Le dispositif, par extension, peut s'adapter aux perfusions utilisant des flacons en verres. Dans ce cas, une prise d'air (4) est à prévoir entre le perforateur (2) et le robinet à trois voies (3).

Le dispositif selon l'invention, en autorisant efficacement l'adjonction d'un produit de rinçage, en toute sécurité, pour le malade et l'infirmière, permet l'évacuation totale du produit actif contenu dans la tubulure (9) assure le rinçage parfait de la veine du patient. Il se destine à la perfusion de produits chimiothérapiques, antibiotiques, anesthésiants ou sanguins pour l'homme, mais aussi, par extension, de produits identiques vétérinaires pour les animaux.

## Revendications

1. Dispositif pour permettre l'injection en toute sécurité de la totalité de produits antimitotiques, antibiotiques ou plaquettes sanguines lors de leur administration en intraveineuse lente comportant une chambre d'air goutte-à-goutte (7), un robinet à trois voies (3) qui est scellé directement sur la chambre d'air (7), un perforateur (2) qui est scellé sur le robinet et une tubulure d'injection (9) qui est reliée au fond de la chambre d'air (7), ledit dispositif étant **caractérisé en ce qu'**un guide vertical (11) est scellé sur le fond (12), en forme de cône, de la chambre d'air (7), une bille flottante (10) étant intégrée dans le guide vertical (11), ladite bille étant adaptée par son diamètre et son poids à obturer un orifice (14) de départ vers la tubulure d'injection (9), au fond de la chambre d'air (7), quand le niveau résiduel d'un liquide à injecter atteint un minimum dans la chambre d'air (7).

2. Dispositif selon la revendication 1 **caractérisé en ce que** le robinet (3) surmonté du perforateur (2) est soudé à la chambre d'air (7) dont il est solidaire et **en ce que** l'une des voies destinée à l'adjonction d'un produit de rinçage (13) est à pas de vis clampé (5) pour permettre d'y visser une seringue, elle aussi, à pas de vis; le clamp (5) empêchant sa désolidarisation.

3. Dispositif selon la revendication 1 ou la revendication 2 **caractérisé en ce qu'**un filtre (8) à résidu est serti, à l'intérieur et au tiers haut de la chambre d'air (7)

4. Dispositif selon l'une quelconque des revendications précédentes **caractérisé en ce que** la tubulure d'injection (9) dépasse d'un à deux millimètres la base intérieure du cône (12), pour permettre à la bille flottante (10) de s'y poser, obturant ainsi hermétiquement la tubulure d'injection (9); cette même bille (10) se remettant à flotter dans la colonne de guidage (11) lors de l'adjonction du produit de rinçage (13).

5. Dispositif selon l'une quelconque des revendications précédentes **caractérisé en ce que** l'orifice (14) épouse la forme arrondie de la base de la bille (10), elle-même d'un poids adapté, pour permettre une étanchéité parfaite.

6. Dispositif selon l'une quelconque des revendications précédentes **caractérisé en ce que** le guidage vertical de la bille flottante (10) est assuré par un tube translucide (11), fermé en son sommet, perforé sur ses parois latérales et soudé à la base interne de l'orifice (14) et d'un diamètre supérieur de deux millimètres de celui de la bille flottante (10); les perforations sur les parois latérales du guide (11) permettant au maximum l'évacuation du produit liquide, et la pose de la bille flottante (10) sur l'orifice (14).

7. Dispositif selon l'une des revendications 1 à 6 **caractérisé en ce qu'**il comporte une poche souple plastique contenant le produit (1) à administrer par intraveineuse lente.

8. Dispositif selon l'une des revendications 1 à 7 **caractérisé en ce qu'**il comprend une prise d'air (4) située entre le perforateur (2) et le robinet à trois voies (3) dans le cas d'une utilisation d'un flacon en verre au lieu d'une poche souple plastique.

## Patentansprüche

1. Vorrichtung zur Ermöglichung der vollkommen sicheren Injektion der Gesamtmenge von Antimitotika, Antibiotika oder Blutplättchen bei ihrer langsamen intravenösen Verabreichung mit einer tropfenweisen Luftkammer (7), einem Drei-Wege-Hahn (3), der direkt auf der Luftkammer (7) versiegelt ist, einer Perforiervorrichtung (2), die auf dem Hahn versiegelt ist und einem Einspritzstutzen (9), der mit dem Boden der Luftkammer (7) verbunden ist, wobei die genannte Vorrichtung **dadurch gekennzeichnet ist, dass** eine vertikale Führung (11) auf dem trichterförmigen Boden (12) der Luftkammer (7) versiegelt ist, wobei eine schwimmende Kugel (10) in die vertikale Führung (11) integriert ist, wobei die genannte Kugel durch ihren Durchmesser und ihr Gewicht zum Verschließen einer Ausgangsöffnung (14) zum Einspritzstutzen (9) am Boden der Luftkammer (7) geeignet ist, wenn das Restniveau einer zu injizierenden Flüssigkeit in der Luftkammer (7) ein Minimum erreicht.

2. Vorrichtung gemäß Anspruch 1, **dadurch gekennzeichnet, dass** der Hahn (3), über dem die Perforiereinrichtung (2) angebracht ist, an der Luftkammer (7) verschweißt ist, mit der er fest befestigt ist und dass eines der zur Verabreichung eines Spülprodukts (13) bestimmten Wege einen geklampten Gewindegang aufweist, um dort die Verschraubung einer Spritze zu ermöglichen, die ebenfalls einen Gewindegang aufweist; wobei der Klamp (5) ihr Ablösen verhindert.

3. Vorrichtung gemäß Anspruch 1 oder 2, **dadurch gekennzeichnet, dass** ein Rückstandsfilter (8) im Innern und auf einem Drittel der Höhe der Luftkammer (7) gefasst ist.

4. Vorrichtung gemäß einem der vorhergehenden Ansprüche, **dadurch gekennzeichnet, dass** der Einspritzstutzen (9) um einen oder zwei Millimeter über die innere Basis des Trichters (12) hervorsteht, damit sich die schwimmende Kugel (10) dort absetzen kann und somit den Einspritzstutzen (9) hermetisch abriegeln; wobei dieselbe Kugel (10) beim Hinzufügen des Spülprodukts (13) wieder in der Führungssäule (11) schwimmt.

5. Vorrichtung gemäß einem der vorhergehenden Ansprüche, **dadurch gekennzeichnet, dass** die Öffnung (14) die abgerundete Form der Basis der Kugel (10) annimmt, die ihrerseits ein geeignetes Gewicht aufweist, um eine perfekte Abdichtung zu ermöglichen.

6. Vorrichtung gemäß einem der vorhergehenden Ansprüche, **dadurch gekennzeichnet, dass** die vertikale Führung der schwimmenden Kugel (10) durch eine an ihrer Spitze geschlossene, an ihren Seitenwänden perforierte und an der inneren Basis der Öffnung (14) festgeschweißte, transparente Röhre (11) mit einem den Durchmesser der schwimmenden Kugel (10) um zwei Millimeter übersteigenden Durchmesser gewährleistet wird; wobei die Perforierungen an den Seitenwänden der Führung (11) maximal den Austrag des flüssigen Produkts und das Aufsetzen der schwimmenden Kugel (10) auf der Öffnung (14) erlauben.

7. Vorrichtung gemäß Anspruch 1 bis 6, **dadurch gekennzeichnet, dass** sie einen das durch langsame intravenöse Verabreichung zu verabreichende Produkt (1) enthaltenden elastischen Plastikbeutel umfasst.

8. Vorrichtung gemäß Anspruch 1 bis 7, **dadurch gekennzeichnet, dass** es eine Luftöffnung (4) zwischen der Perforiervorrichtung (2) und dem Drei-Wege-Hahn (3) für den Fall eines Einsatzes eines Glasflakons anstelle eines elastischen Plastikbeutels umfasst.

## Claims

1. A device for allowing the injection in complete safety of all antimitotic, antibiotic or blood platelet products during their slow intravenous administration, comprising a drop by drop air chamber (7), a three-way valve (3) which is directly sealed on the air chamber (7), a perforator (2) which is sealed on the valve and an injection tube (9) which is connected to the bottom of the air chamber (7), the said device being **characterised in that** a vertical guide (11) is sealed on the cone-shaped bottom (12) of the air chamber (7), a floating ball (10) being integrated in the vertical guide (11), the said ball being adapted, through its diameter and weight, to close off an outlet orifice (14) to the injection tube (9), at the bottom of the air chamber (7), when the residual level of a liquid to be injected reaches a minimum in the air chamber (7).

2. A device according to Claim 1, **characterised in that** the valve (3) surmounted by the perforator (2) is welded to the air chamber (7) to which it is fixed and **in that** one of the ways intended for the addition of a rinsing product (13) has a screw thread clamped (5) so as to enable a syringe to be screwed onto it, it too with a screw thread; the clamp (5) preventing its disconnection.

3. A device according to Claim 1 or Claim 2, **characterised in that** a residue filter (8) is clamped inside and one third of the way up the air chamber (7).

4. A device according to any one of the preceding claims, **characterised in that** the injection tube (9) projects by one to two millimetres beyond the internal base of the cone (12), to enable the floating ball (10) to rest there, thus hermetically closing off the injection tube (9); this same ball (10) recommencing floating in the guide column (11) when the rinsing product (13) is added.

5. A device according to any one of the preceding claims, **characterised in that** the orifice (14) matches the rounded shape of the base of the ball (10), itself of an adapted weight, to allow a perfect seal.

6. A device according to any one of the preceding claims, **characterised in that** the vertical guidance of the floating ball (10) is provided by a translucent tube (11), closed at its top, perforated on its side walls and welded to the internal base of the orifice (10), and with a diameter two millimetres greater than that of the floating ball (10); the perforations on the side walls of the guide (11) allowing discharge of the liquid product to the maximum possible extent and the positioning of the floating ball (10) on the orifice (14).

7. A device according to one of Claims 1 to 6, **characterised in that** it comprises a flexible plastic bag containing the product (1) to be slowly administered intravenously.

8. A device according to one of Claims 1 to 7, **characterised in that** it comprises a breather (4) situated between the perforator (2) and the three-way valve (3) in the case of the use of a glass vial instead of a flexible plastic bag.
